# EUROPEAN PATENT SPECIFICATION

(11) **EP 1 529 766 B1**
(45) Date of publication and mention of the grant of the patent: **01.02.2006**
(21) Application number: 04025494.8
(22) Date of filing: 27.10.2004
(51) Int. Cl.: C05F 17/00, C05F 5/00, C12N 1/00, C12N 1/08, C12N 15/10

(54) **Process for fragmentation of fungal, bacterial or yeast cell DNA and for inactivating residual antibiotics in fermentation biomass**
Verfahren zur Fragmentierung von DNS aus Pilzen, Bakterien oder Hefen und zur Inaktivierung von verbliebenen Antibiotika in einer Fermentationsbiomasse
Procédé pour la fragmentation d'ADN de champignons, bactéries et levures et pour l'inactivation de restes d'antibiotiques dans une biomasse de fermentation

(30) Priority: 05.11.2003 IT MI20032129
(43) Date of publication of application: 11.05.2005
(73) Proprietor: ACS DOBFAR S.p.A., 20067 Tribiano (Milano) (IT)
(72) Inventor: Archilletti, Tiziana, 03100 Frosinone (IT); Morlupi, Alessandro, 00156 Roma (IT); Sequi, Paolo, 00184 Roma (IT); Nannipieri, Paolo, 57100 Livorno (IT); Pietramellara, Giacomo, 50134 Firenze (IT); Mocali, Stefano, 50139 Firenze (IT)
(74) Representative: Frignoli, Luigi

(56) References cited:
- WO-A-98/07670
- GB-A- 2 285 043
- ANDERSEN JENS TONNE ET AL: "Using inactivated microbial biomass as fertilizer: The fate of antibiotic resistance genes in the environment" RESEARCH IN MICROBIOLOGY, vol. 152, no. 9, November 2001 (2001-11), pages 823-833, XP002308722 ISSN: 0923-2508

## Description

The present invention relates to a process for fragmentation of fungal, bacterial or yeast cell DNA and for inactivating any residual antibiotics directly in fermentation biomass.

Those fermentations which lead to the formation of antibiotics are known to produce aqueous suspensions of fungal or bacterial cells which, when filtered off and removed from the respective aqueous solution rich in fermented antibiotic principle, give biomass which represents a waste substance the disposal of which still involves considerable expense affecting the cost of the antibiotic produced.

Likewise, in fermentation using yeast (for example for alcoholic beer fermentation) biomass is produced which has to be disposed of.

The said biomass is rich in nitrogen, phosphorus, potassium, carbon and organic compounds, rendering it ideal for transformation into fertilizer, such mass being bioavailable for ready assimilation by soil microflora and having no undesirable ground-contaminating substances. In practice, the high percentage of organic-biological components makes the biomass the ideal fertilizer for soil intended for those organic crops increasingly required by the fruit and vegetable market.

However, the residual biomass from the said fermentations contains potentially transgenic genetic material which, if deriving from antibiotic production fermentation, also contains residual antibiotics the presence of which in a fertilizer could damage, modify or alter the soil microflora.

The potentially transgenic material is formed from the double helix of the fungal, bacterial or yeast DNA which could give rise to the formation of genetically modified organisms (GMO). The use of biomass which contains it is hence prohibited by the most advanced European health regulations.

It is however known from the literature that if the size of a potentially transgenic base pair fragment deriving from DNA fragmentation comprises a base pair sequence of less than 1000 units, it is no longer considered a gene and is consequently no longer able to create interference with the soil microflora and produce genetically modified structures. See for example:
- Michaela Pertea et al.; "Bioinformatics: Finding Genes in Plasmodium Falciparum", Nature, 02 March 2000; and
- "Size Limits of Very Small Microorganisms": Proceedings of a Workshop 1999, page 164 - Steering Group for the Workshop, National Research Council.

There are currently no known industrially applicable processes for reducing to below 1000 units the base pair sequences relating to the DNA present in residual biomass from fermentation processes. This can be demonstrated by the fact that the fungal biomass obtained by an important European company as fermentation residue in Cephalosporin C production is used as microflora in many countries, in spite of the presence of potentially transgenic material therein. This is against the regulations of the European Community, in open violation of a law current in Italy.

An analytical method able to quantify with high precision the number of base pair sequences (i.e. the dimensions of the extracted fragments) with reference to DNA after treatment, has been devised by an important Italian Public Research Institute. This method was verified and officially validated with its publication in the Italian Official Gazette of 21.05.2003, General Series 116. It is therefore not necessary to explain this method as it is described in detail in the aforesaid publication which officializes it.

Residual antibiotic in fermentation biomass can be easily monitored, until its disappearance, by well known high pressure liquid chromatography (HPLC), which requires no detailed explanation.

The main object of the present invention is therefore to provide a process of simple and reliable implementation on an industrial scale which both enables the double helix of fungal, bacterial or yeast DNA present in fermentation biomass to be fragmented to below 1000 base pairs, and enables any antibiotic residue in said biomass to be eliminated.

This and further objects are attained by a process by which the fermentation biomass containing potentially transgenic genetic material and possible antibiotic residue is firstly acidified to pH within the range 5.0-1.0 with aqueous acid solutions, then heated to a temperature between +50 and 110°C, and finally cooled to ambient temperature and neutralized with organic and/or inorganic bases.

As the process consists of a treatment which destroys both the DNA and any residual antibiotic, operative parameters such as the agitation rate during heating and acid addition modalities are of little importance in attaining the stated object. It has however been noted that heating must be continued for a longer time if the temperature is low (close to +50°C) than if the temperature is high (close to +110°C), to enable the heat administered at acid pH to act on the entire mass, so contributing to hydrolyze amides and a large part of the DNA and antibiotic esters: in particular, in penicillins and cephalosporins the thermolability of the amide bond at acid pH favours inactivation of the residual antibiotic active principle, with opening of the betalactam ring, on the basis of the heating time, which is variable according to the stability of the antibiotic subjected to inactivation treatment.

The required heating time duration can be easily verified by analyzing the treated biomass by the aforesaid methods, with regard both to the dimensions of the extracted and analyzed DNA fragments and the presence of residual antibiotic.

Preferably the temperature used in the process of the invention is between +80° and +100°C; the preferred acids used in aqueous solution are those of the group comprising acetic acid, methanesulphonic acid, phosphoric acid and sulphuric acid (which are compatible with the presence of acetates, phosphates and sulphates in agricultural soil); while neutralization is effected with bases chosen from the group consisting of calcium, sodium, potassium, ammonium hydroxide, or triethylamine, diethylamine and similar organic bases.

Even if effective, those acids which can give rise to the formation of salts incompatible with the fertilizing properties of a fertilizer cannot be used, such as hydrochloric acid which while achieving the stated object would lead to the undesirable formation of chlorides to be added to the soil.

The following non-limiting examples illustrate the modalities of implementing the process of the present invention.

### EXAMPLE 1

A fungal biomass resulting from a standard industrial fermentation of Cephalosporin C was acidified to pH 1.0 with 96% H₂SO₄. The acidified mass was maintained heated to 90°C for 90 min, then cooled to ambient temperature and neutralized to pH 7 with Ca(OH)₂.

The final mass obtained was analyzed by the method described in detail in the Official Italian Gazette of 21.05.2003 (aforestated), showing only the residual presence of fungal DNA fragments below 1000 base pairs.

### EXAMPLE 2

Proceeding as in Example 1 but acidifying to pH 1.0 with H₃PO₄ and heating to 90°C for 60 min, the test for DNA showed the absence of fragments greater than 500 base pairs.

### EXAMPLE 3

Proceeding as in Example 1 but acidifying firstly to pH 2.0 with a concentrated solution of H₃PO₄ then adjusting the pH to 1.0 with an aqueous 30% H₂SO₄ solution, and then heating to 90°C for 60 min, no DNA fragments greater than 500 base pairs were observed.

### EXAMPLE 4

Operating as in Example 3 on a sample of fungal biomass from Cephalosporium acremonium grown in a flask, after removing 50% supernatant liquid from the biomass (which is hence more than 50% concentrated), results similar to those indicated in Example 3 were obtained.

### EXAMPLE 5

500 ml of biomass from an industrial fermentation of Cephalosporin C (spent liquor), withdrawn from the plant process, were treated with 60% phosphoric acid to adjust the pH from 3.1 to 2.0, which was then further lowered to 1.0 with sulphuric acid. The suspension obtained was maintained at 100°C for 300 min. After treatment, the sample was cooled to 20-25°C and neutralized to pH 7.0 with potassium hydroxide. A portion of this suspension was filtered, the solution obtained then being tested for the presence of residual Cephalosporin C by HPLC. The antibiotic was less than the detection limit (i.e. <0.9 ppm). After treatment, determination of residual DNA shows fragments all below 1000 base pairs.

Results for DNA and residual antibiotic similar to the aforestated are obtained if the sample is heated to 110°C for 60 min.

### EXAMPLE 6

500 ml of biomass of Cephalosporin C (spent liquor), withdrawn from an industrial process, were treated with 30% sulphuric acid to adjust the pH from 3.1 to 1.0. The suspension obtained is heated and neutralized as in Example 5.

Checked for residual antibiotic, the sample shows no presence of residual Cephalosporin C (<0.9 ppm), with DNA fragments of less than 1000 base pairs.

### EXAMPLE 7

A sample of spent mycelium (5 litres of fungal biomass) withdrawn on termination of fermentation from an industrial Cephalosporin C process, with residual Cephalosporin C = 1.4 g/l, was transferred to a 10 litre boiler of AISI 316 steel fitted with a stirrer, heating jacket, manhole thermometer and pH meter. 1 litre of water was added to the sample to increase its fluidity and improve stirring.

60 ml of 96% H₂SO₄ were added to the mass while stirring over a time of 12 min to adjust the pH from 3.43 initial to 1.02 final.

While stirring, the suspension was heated to 100°C over 35 min and maintained at that temperature for 360 min. The suspension was then cooled to 23°C, topped up with the water lost by evaporation (about 400 ml), and neutralized to pH 7.03 by adding triethylamine. The sample treated in this manner was then checked for the presence of DNA and residual antibiotic. DNA fragments of less than 1000 base pairs and residual Cephalosporin C of less than 1 ppm were found.

### EXAMPLE 8

A sample of spent mycelium as in Example 7 was acidified to pH 1.01 with 230 ml of 85% phosphoric acid. The sample was then heated to 90°C for 300 min, cooled to ambient temperature, topped up to its initial volume, neutralized to pH 7.1 with diethylamine and analyzed for DNA and residual Cephalosporin C. Values comparable to those of Example 7 were found.

### EXAMPLE 9

A sample of spent mycelium as in Example 8 was acidified to pH 2.02 with 56 ml of 85% H₃PO₄ and then adjusted to pH 1.02 with 50 ml of 96% H₂SO₄. The sample was heated to 95°C for 180 min, cooled to ambient temperature, topped up to its initial volume and neutralized to pH 7.04 with Ca(OH)₂. Analyses for DNA and residual antibiotic gave results comparable to those of Example 7.

### EXAMPLE 10

A 3000 g sample of biomass (spent bacterial cells), filtered off on termination of fermentation in tylosin production, was diluted with water to a stirrable volume (6 litres). The diluted sample was transferred to a 10 litre AISI 316 steel boiler equipped as in Example 7. The sample, of pH 5.11, was adjusted to pH 1.04 by adding 54 ml of 96% H₂SO₄. The resultant suspension was heated to 95°C for 360 min, cooled to ambient temperature, topped up to its initial volume by adding water (about 350 ml) and neutralized to pH 7.09 with 25% NH₄OH. DNA and residual antibiotic analyses confirm the absence of base pairs > 500, with antibiotic < 1 ppm.

A sample such as that described in this example was adjusted to pH 2.2 with 85% H₃PO₄ and heated to 100°C for 450 min. The sample, cooled and neutralized with Ca(OH)₂, was analyzed for DNA and residual antibiotic, obtaining values comparable to the aforestated.

An identical sample was acidified to pH 1.32 with 90% methanesulphonic acid; the suspension obtained was kept stirring at 95°C for 420 min. cooled and neutralized with calcium hydroxide.. Analysis showed that it did not contain DNA fragments > 500 base pairs nor tylosin in > 1 ppm concentration.

In the same manner a similar sample of biomass was acidified to pH 4.01 with CH₃COOH giving a suspension which was kept stirring at 95°C for 590 min, cooled and neutralized with NH₄OH and then analyzed for DNA and residual antibiotic, giving results comparable to those indicated in the initial part of this example.

The aforegiven examples describe the treatment for inactivating DNA and residual antibiotic in fungal and bacterial biomass from Cephalosporin C and Tylosin production. The same treatment can be implemented on fermentation masses using yeast or other fungal or bacterial strains used to ferment other antibiotic principles (such as Penem, Penam, Cepham, Cephem, Tetracyclines, Macrolides, Aminoglycosides).

## Claims

1. A process for fragmentation of fungal, bacterial or yeast cell DNA and for inactivating antibiotic residues in fermentation biomass, **characterised by** firstly acidifying the biomass to pH between 5.0 and 1.0 with aqueous acid solutions, then heating it to a temperature between +50°C and +110°C, then cooling it to ambient temperature and finally neutralizing it with organic and/or inorganic bases.

2. A process as claimed in claim 1, **characterised in that** the acids used to acidify said aqueous solutions are chosen from the group consisting of acetic acid, methanesulphonic acid, phosphoric acid and sulphuric acid.

3. A process as claimed in claims 1 and 2, **characterised in that** said heating is carried out to a temperature between +80°C and +100°C.

4. A process as claimed in claims 1-3, **characterised in that** said neutralization is effected with bases chosen from the group consisting of calcium, sodium, potassium, ammonium hydroxide, triethylamine, diethylamine and similar organic bases.

## Patentansprüche

1. Verfahren zur Fragmentierung von Pilz-, Bakterien- oder Hefezell-DNS und zum Inaktivieren von antibiotischen Rückständen in einer Fermentationsbiomasse, **gekennzeichnet durch** erstens Ansäuern der Biomasse auf einen pH zwischen 5,0 und 1,0 mit wässrigen Säurelösungen, dann Erhitzen auf eine Temperatur zwischen +50 °C und +110 °C, dann Kühlen auf Umgebungstemperatur und schließlich Neutralisieren mit organischen und/oder anorganischen Basen.

2. Verfahren gemäß Anspruch 1, **dadurch gekennzeichnet, dass** die Säuren, die zum Ansäuern der wässrigen Lösungen verwendet werden, aus der Gruppe, bestehend aus Essigsäure, Methansulfonsäure, Phosphorsäure und Schwefelsäure, ausgewählt sind.

3. Verfahren gemäß den Ansprüchen 1 und 2, **dadurch gekennzeichnet, dass** das Erhitzen bis zu einer Temperatur zwischen +80 °C und +100 °C ausgeführt wird.

4. Verfahren gemäß den Ansprüchen 1-3, **dadurch gekennzeichnet, dass** die Neutralisation mit Basen bewirkt wird, ausgewählt aus der Gruppe, bestehend aus Calcium-, Natrium-, Kalium-, Ammoniumhydroxid, Triethylamin, Diethylamin und ähnlichen organischen Basen.

## Revendications

1. Procédé pour la fragmentation d'ADN de champignons, bactéries ou de levures et pour l'inactivation de résidus antibiotiques dans une biomasse de fermentation, **caractérisé** d'abord par une acidification de la biomasse à un pH entre 5,0 et 1,0 avec des solutions acides aqueuses, puis par son chauffage à une température entre +50 °C et +110°C, puis par son refroidissement à température ambiante et enfin par sa neutralisation à l'aide de bases organiques et/ou inorganiques.

2. Procédé selon la revendication 1, **caractérisé en ce que** les acides utilisés pour acidifier lesdites solutions aqueuses sont choisis dans le groupe comprenant l'acide acétique, l'acide méthanesulfonique, l'acide phosphorique et l'acide sulfurique.

3. Procédé selon la revendication 1 et la revendication 2, **caractérisé en ce que** ledit chauffage est réalisé à une température entre +80 °C et +100 °C.

4. Procédé selon les revendications 1 à 3, **caractérisé en ce que** ladite neutralisation est réalisée avec des bases choisies dans le groupe comprenant le calcium, le sodium, le potassium, l'hydroxyde d'ammonium, la triéthylamine, la diéthylamine et des bases organiques similaires.
